# EUROPÄISCHE PATENTSCHRIFT

(11) **EP 3 038 708 B1**
(45) Veröffentlichungstag und Bekanntmachung des Hinweises auf die Patenterteilung: **30.08.2017**
(21) Anmeldenummer: 14755008.1
(22) Anmeldetag: 17.07.2014
(51) Int. Cl.: A61Q 5/08, A61Q 5/10, A61K 8/81, A61K 8/90, A61K 8/92, A61K 8/22, A61K 8/23

(54) **BLONDIERMITTEL MIT FETTALKOHOLEN**
COMPOSITION FOR BLEACHING HAIR WITH FATTY ALCOHOLS
COMPOSITION POUR LA BLONDAGE DES CHEVEUX À BASE DES ALCOHOLS GRASSES

(30) Priorität: 28.08.2013 DE 102013217204
(43) Veröffentlichungstag der Anmeldung: 06.07.2016
(73) Patentinhaber: Henkel AG & Co. KGaA, 40589 Düsseldorf (DE)
(72) Erfinder: MÜLLER, Burkhard, 40221 Düsseldorf (DE); ERKENS, Udo, 41468 Neuss-Grimlinghausen (DE)
(86) Internationale Anmeldenummer: PCT/DE2014/200327
(87) Internationale Veröffentlichungsnummer: WO 2015/028005

(56) Entgegenhaltungen:
- EP-A1- 1 034 777
- EP-A1- 1 380 287
- EP-A1- 1 430 875
- US-A1- 2003 190 297

## Beschreibung

Die vorliegende Erfindung betrifft Mittel zur oxidativen Farbveränderung im Bereich der Kosmetik, die sich besonders zum Aufhellen keratinischer Fasern, insbesondere menschlicher Haare, eignen.

Die in Blondiermitteln enthaltenen Oxidationsmittel sind in der Lage, die Haarfaser durch die oxidative Zerstörung des haareigenen Farbstoffes Melanin aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt.

Aus Stabilitätsgründen werden handelsübliche Blondiermittel gewöhnlich in zwei getrennt voneinander verpackten Zubereitungen angeboten, die unmittelbar vor der Anwendung zu einer fertigen Anwendungszubereitung vermischt werden. Üblicherweise bestehen handelsübliche Blondiermittel aus einer flüssigen Oxidationsmittelzubereitung und einem Pulver, das feste Oxidationsmittel enthält. Alternativ können anstelle des Pulvers pastenförmige Mittel mit einer flüssigen Oxidationsmittelzubereitung vermischt werden, wodurch die Problematik des Staubens bei der Herstellung und beim Vermischen vermieden wird. Produkte mit weiteren Komponenten werden ebenfalls im Handel angeboten.

Pastenförmige Blondiermittel enthalten meist größere Mengen eines inerten Öls, was zu Stabilitätsproblemen (Absetzen der festen Oxidationsmittel vom Öl) führen kann. Selbst bei noch nicht vollständig abgesetzten Peroxidisulfaten kann ein Konzentrationssgradient innerhalb der Verpackung auftreten, so dass verschiedene Portionen aus der Verpackung nach dem Vermischen unterschiedliche Aufhellung bewirken können. Um diese Probleme zu minimieren, ist eine hohe Viskosität wünschenswert.

Auf der anderen Seite muß die Viskosität der Blondierpaste so gering sein, dass sie sich gut und schnell mit der flüssigen Oxidationsmittelzubereitung vermischen läßt. Die resultierende Blondiermischung muß darüber hinaus flüssig genug sein, um sich leicht und gleichmäßig applizieren zu lassen, aber dickflüssig genug, um nicht vom Kopf oder von Anwendungshilfsmitteln wie Pinseln herabzutropfen. Zusätzlich sollte sich auch die resultierende Blondiermischung nicht trennen, da Absetzen oder Phasenseparationen vom Kunden als Qualitätsmangel wahrgenommen werden.

Die WO 2009/134875 A1 beschreibt Blondiermittel, welche Persulfatsalze und ein Ölgel enthalten, das seinerseits aus Öl(en) und bestimmten Polymeren zusammengesetzt ist.
Als wünschenswerte Eigenschaften des Mittels gemäß dieser Erfindung sind Stabilität gegen Absetzen und Phasentrennung angegeben.

Die EP 1 034 777 A1 offenbart Mittel zum Aufhellen keratinischer Fasern, welche mindestens zwei getrennt voneinander verpackte Zubereitungen (A) und (B) enthalten, die unmittelbar vor der Anwendung zu einer Anwendungsmischung vermischt werden, wobei die Zubereitungen (A) ölbasiert sind und Polymer(e) enthalten, welche Oleo- bzw. Lipogele ausbilden. Dabei werden auch Mischungen mit natürlichen Polymeren, beispielsweise Xanthan, offenbart.

Aufgabe der vorliegenden Erfindung war es, die Eigenschaften von Blondiermitteln weiter zu verbessern. dabei sollte insbesondere die Lagerstabilität erhöht werden, wobei nicht nur die physikalische Stabilität (Absetzen, Phasenseparation), sondern auch die chemische Stabilität (Abbau der Persalze) verbessert werden sollte.

Es hat sich gezeigt, dass höher ölhaltige und mit speziellen Polymeren verdickte Blondierpasten dann besonders stabil sind, wenn die Pasten zusätzlich bestimmte Fettalkohole enthalten.

Gegenstand der vorliegenden Erfindung sind in einer ersten Ausführungsform Mittel zum Aufhellen keratinischer Fasern, enthaltend - bezogen auf ihr Gewicht -
a) 20 bis 75 Gew.-% Öl(e);
b) 0,05 bis 5 Gew.-% Xanthan,
c) 1 bis 70 Gew.-% Peroxidisulfat(e),
d) 1 bis 15 Gew.-% Cetearylakohol.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Als ersten Inhaltsstoff enthalten die erfindungsgemäßen Mittel ein oder mehrere Öl(e). Vorzugsweise ist/sind diese(s) Öl(e) unter Normalbedingungen flüssig.
Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 10 - 12000 Pa (0,1 - 90 mm Hg), besonders bevorzugt 13 - 3000 Pa, außerordentlich bevorzugt 15 - 500 Pa, aufweisen.
Flüchtige kosmetische Öle sind üblicherweise unter cyclischen Siliconölen mit der INCI-Bezeichnung Cyclomethicone ausgewählt. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.
Ein erfindungsgemäß bevorzugter Cyclomethicone-Ersatzstoff ist eine Mischung aus C₁₃-C₁₆-Iso-paraffinen, C₁₂ - C₁₄-Isoparaffinen und C₁₃ - C₁₅-Alkanen, deren Viskosität bei 25°C im Bereich von 2 bis 6 mPas liegt und die einen Dampfdruck bei 20°C im Bereich von 10 bis 150 Pa, bevorzugt 100 bis 150 Pa, aufweist. Eine solche Mischung ist z. B. unter der Bezeichnung SiClone SR-5 von der Firma Presperse Inc. erhältlich.
Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan (L₂), Octamethyltrisiloxan (L₃), Decamethyltetrasiloxan (L₄), wie sie z. B. in den Handelsprodukten DC 2-1184, Dow Corning^{®} 200 (0,65 cSt) und Dow Corning^{®} 200 (1,5 cSt) von Dow Corning enthalten sind, und niedermolekulares Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.
Weitere bevorzugte erfindungsgemäße Produkte enthalten mindestens ein flüchtiges Nichtsiliconöl. Bevorzugte flüchtige Nichtsiliconöle sind ausgewählt aus C₈-C₁₆-Isoparaffinen, insbesondere aus Isononan, Isodecan, Isoundecan, Isododecan, Isotridecan, Isotetradecan, Isopentadecan und Isohexadecan, sowie Mischungen hiervon. Bevorzugt sind C₁₀-C₁₃-Isoparaffin-Mischungen, insbesondere solche mit einem Dampfdruck bei 20°C von 10 - 400 Pa, bevorzugt 13 - 100 Pa.
Als kosmetisches Öl erfindungsgemäß weiterhin besonders bevorzugt sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isodecylneopentanoat, Isononylisononanoat, 2-Ethylhexylpalmitat und 2-Ethylhexylstearat. Ebenfalls bevorzugt sind Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat, Ethylenglycoldipalmitat. n-Hexyllaurat, n-Decyloleat, Oleyloleat, Oleylerucat, Erucyloleat, C₁₂-C₁₅-Alkyl-lactat und Di-C₁₂-C₁₃-Alkylmalat sowie die Benzoesäureester von linearen oder verzweigten C₈₋₂₂-Alkanolen. Besonders bevorzugt sind Benzoesäure-C₁₂-C₁₅-Alkylester, z. B. erhältlich als Handelsprodukt Finsolv^{®} TN (C₁₂-C₁₅-Alkylbenzoat), sowie Benzoesäureisostearylester, z. B. erhältlich als Finsolv^{®} SB, 2-Ethylhexylbenzoat, z. B. erhältlich als Finsolv^{®} EB, und Benzoesäure-2-octyldodecylester, z. B. erhältlich als Finsolv^{®} BOD.
Als besonders vorteilhaft hat sich der Einsatz von Isopropylestern von C₁₂-C₁₈-Carbonsäuren, insbesondere der Einsatz von Isopropylmyristat, und besonders bevorzugt Mischungen von Isopropylmyristat mit C₁₀-C₁₃-Isoparaffin-Mischungen, letztere bevorzugt mit einem Dampfdruck bei 20°C von 10 - 400 Pa, erwiesen.
Ein weiteres besonders bevorzugtes Esteröl ist Triethylcitrat. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan, Isotetradecan, Isopentadecan und Isohexadecan sowie Mischungen dieser Isoparaffine. Weitere erfindungsgemäß bevorzugte Produkte enthalten Triethylcitrat und mindestens ein C₈-C₁₆-Isoparaffin, ausgewählt aus Isononan, Isodecan, Isoundecan, Isododecan, Isotridecan sowie Mischungen dieser C₈-C₁₆-Isoparaffine. Weitere erfindungsgemäß bevorzugte Produkte enthalten Triethylcitrat und eine Mischung aus Isodecan, Isoundecan, Isododecan und Isotridecan.
Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten C₈₋₃₀-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol^{®} 318 oder Myritol^{®} 331 (BASF/ Cognis) mit unverzweigten Fettsäureresten sowie Glyceryltriisostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in dem erfindungsgemäßen Produkt aus. Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexyl-succinat und Di-(2-hexyldecyl)-succinat.

Weitere erfindungsgemäß besonders bevorzugte nichtflüchtige Nichtsiliconöle sind ausgewählt aus den symmetrischen, unsymmetrischen oder cyclischen Estern der Kohlensäure mit C₆-C₂₀-Alkoholen, z. B. Di-n-caprylylcarbonat (Cetiol^{®} CC) oder Di-(2-ethylhexyl)carbonat (Tegosoft DEC). Ester der Kohlensäure mit C₁-C₅-Alkoholen, z. B. Glycerincarbonat oder Propylencarbonat, sind hingegen keine als kosmetisches Öl geeigneten Verbindungen.
Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Besonders bevorzugt beträgt das Gesamtgewicht an Dimerfettsäureestern 0,5 - 10 Gew.-%, bevorzugt 1 - 5 Gew.-%, jeweils bezogen auf das Gewicht der gesamten Wasser-in-Öl-Emulsion, ohne das Gewicht des Treibmittels zu berücksichtigen.
Weitere kosmetische Öle, die erfindungsgemäß besonders bevorzugt sind, sind ausgewählt aus nicht-flüchtigen Siliconölen. Erfindungsgemäß bevorzugte nichtflüchtige Siliconöle sind ausgewählt aus linearen Polyalkylsiloxanen mit einer kinematischen Viskosität bei 25°C von mindestens 5 cSt bis 2000 cSt, insbesondere ausgewählt aus linearen Polydimethylsiloxanen mit einer kinematischen Viskosität bei 25°C von 5 cSt bis 2000 cSt, bevorzugt 10 bis 350 cSt, besonders bevorzugt 50 - 100 cSt, wie sie z. B. unter den Handelsnamen Dow Corning^{®} 200 bzw. Xiameter PMX von Dow Corning bzw. Xiameter erhältlich sind. Weitere bevorzugte nichtflüchtige Siliconöle sind Phenyltrimethicone mit einer kinematischen Viskosität bei 25°C von 10 bis 100 cSt, bevorzugt von 15 - 30 cSt sowie Cetyldimethicone.
Erfindungsgemäß bevorzugte Mittel enthalten mindestens ein nichtflüchtiges Siliconöl, das bevorzugt ausgewählt ist aus linearen Polyalkylsiloxanen mit einer kinematischen Viskosität bei 25°C von 5 cSt - 2000 cSt, bevorzugt 10 - 350 cSt, besonders bevorzugt 50 - 100 cSt, insbesondere ausgewählt aus linearen Polydimethylsiloxanen mit einer kinematischen Viskosität bei 25°C von 5 cSt - 2000 cSt, bevorzugt 10 - 350 cSt, besonders bevorzugt 50 - 100 cSt, in einer Gesamtmenge von 0,1 - 30 Gew.-%, bevorzugt 1 - 24 Gew.-%, besonders bevorzugt 2 - 18 Gew.-%, außerordentlich bevorzugt 4 - 10 Gew.-%, jeweils bezogen auf das Gewicht des gesamten Mittels.

Von den genannten Ölen haben sich einige als besonders geeignet erwiesen, da sie die physikalische und chemische Stabilität der Blondiermittelpasten über lange Zeiträume garantieren und mit den weiteren erfindungsgemäßen Inhaltsstoffen hervorragend verträglich sind. Erfindungsgemäß bevorzugte Mittel sind dadurch gekennzeichnet, dass sie 22,5 bis 70 Gew.-%, vorzugsweise 25 bis 65 Gew.-%, weiter bevorzugt 27,5 bis 60 Gew.-%, besonders bevorzugt 30 bis 55 Gew.-% und insbesondere 32,5 bis 50 Gew.-% Öl(e) aus der Gruppe Paraffinöl, Polyisobuten, der Alkylbenzoate, Isopropylpalmitat, Isohexadekan, Isododecan, Isononyl-Isononanoat enthalent.

Weiter bevorzugte erfindungsgemäße Mittel enthalten 20 bis 60 Gew.-%, vorzugsweise 22,5 bis 55 Gew.-%, weiter bevorzugt 25 bis 50 Gew.-%, besonders bevorzugt 27,5 bis 45 Gew.-% und insbesondere 30 bis 40 Gew.-% Paraffinöl.

Als weiteren Inhaltstoff enthalten die erfindungsgemäßen Mittel 0,05 bis 5 Gew.-% Xanthan. Erfindungsgemäß bevorzugt sind solche Xanthane, die nach der Quellung transparente Zubereitungen ergeben. Insbesondere bevorzugt ist die Verwendung des Xanthan-Biopolymers, welches unter dem Handelsnamen Keltrol CG-SFT der Firma Kelco vertrieben wird.
In einer bevorzugten Ausführungsform enthält ein erfindungsgemäßes Mittel 0,1 bis 5 Gew.-%, vorzugsweise 0,5 bis 4 Gew.-%, weiter bevorzugt 1 bis 3 Gew.-%, besonders bevorzugt 1,25 bis 2,5 Gew.-% und insbesondere 1,5 bis 2 Gew.-% Xanthan.

Als weiteren wesentlichen Inhaltsstoff enthalten die erfindungsgemäßen Zubereitungen 1 bis 70 Gew.-% Peroxidisulfat(e), wobei bevorzugte erfindungsgemäße Mittel - bezogen auf ihr Gewicht - 5 bis 43,5 Gew.-%, vorzugsweise 10 bis 43 Gew.-%, weiter bevorzugt 15 bis 42,5 Gew.-%, besonders bevorzugt 20 bis 42 Gew.-% und insbesondere 30 bis 41 Gew.-% Kaliumperoxidisulfat enthalten.

Es ist äußerst bevorzugt, die Menge an Kaliumperoxidisulfat immer deutlich größer zu halten als die Menge an eventuell eingesetztem Natrium- und Ammoniumperoxidisulfat. Es hat sich gezeigt, dass mit steigendem Kaliumperoxidisulfat-Anteil an der Gesamtmenge an Peroxidisulfaten die chemische und physikalische Stabilität der erfindungsgemäßen Mittel steigt. In bevorzugten Mitteln liegt daher das Gewichtsverhältnis von Kaliumperoxidisulfat zu Natrium- und Ammoniumperoxidisulfat bei > 2, vorzugsweise bei > 5, weiter bevorzugt bei > 10, noch weiter bevorzugt bei > 15 und insbesondere bei > 20. Dieses Gewichtsverhältnis wird ermittelt, indem die Gew.-%-Menge an Kaliumperoxidisulfat durch die Summe der Gew.-%-Mengen an Natrium- und Ammoniumperoxidisulfat dividiert wird.

Erfindungsgemäß bevorzugte Mittel sind dadurch gekennzeichnet, dass das Gewichtsverhältnis von im Mittel enthaltenem Kaliumperoxidisulfat zu im Mittel enthaltenen Natrium- und Ammonium-Peroxidisulfaten > 10:1, vorzugsweise > 12,5 :1, weiter bevorzugt > 15:1, besonders bevorzugt > 17,5:1 und insbesondere > 20:1 beträgt.

Äußerst bevorzugte erfindungsgemäße Mittel enthalten 0 bis < 2,5 Gew.-%, vorzugsweise 0 bis < 1 Gew.-%, weiter bevorzugt 0 bis < 0,5 Gew.-%, besonders bevorzugt 0 bis < 0,1 Gew.-% und insbesondere 0 Gew.-% Peroxidisulfate aus der Gruppe Natriumperoxidisulfat und/oder Ammoniumperoxidisulfat.

Als weiteren wesentlichen Inhaltsstoff enthalten die erfindungsgemäßen Zubereitungen 1 bis 15 Gew.-% Cetearylakohol. Dieser ist zur physikalischen und chemischen Stabilisierung besonders geeignet. Besonders bevorzugte Mittel enthalten 1 bis 15 Gew.-%, vorzugsweise 2 bis 10 Gew.-%, weiter bevorzugt 3,5 bis 8 Gew.-%, besonders bevorzugt 4 bis 7 Gew.-% und insbesondere 5 bis 6,5 Gew.-% Cetearylalkohol.

Die physikalische Stabilität der erfindungsgemäßen Mittel kann durch den Einsatz weiterer Konsistenzgeber noch gesteigert werden. Hier bieten sich insbesondere längerkettige Fettalkohole an, deren Einsatz im Gegensatz zum Cetearylakohol optional ist.
Als Konsistenzgeber können die erfindungsgemäßen Mittel bevorzugt langkettige Fettalkohole enthalten, die vorzugsweise aus der Gruppe Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13*E*)-Docosen-1-ol) ausgewählt sind.
Diese langkettigen Fettalkohole besitzen ein Kettenlänge von mindestens 20 C-Atomen. Innerhalb dieser Gruppe haben sich spezielle langkettige Fettalkohole als ganz besonders geeignet erwiesen.
In einer besonders bevorzugten Ausführungsform ist ein Mittel zum Blondieren und/oder Bleichen von keratinischen Fasern dadurch gekennzeichnet, dass es Arachylalkohol (Eicosan-1-ol) enthält.
In einer weiteren besonders bevorzugten Ausführungsform ist en Mittel zum Blondieren und/oder Bleichen von keratinischen Fasern dadurch gekennzeichnet, dass es Behenylalkohol (Docosan-1-ol) enthält.
In einer weiteren besonders bevorzugten Ausführungsform ist ein Mittel zum Blondieren und/oder Bleichen von keratinischen Fasern dadurch gekennzeichnet, dass es Arachylalkohol (Eicosan-1-ol) und/oder Behenylalkohol (Docosan-1-ol) enthält.
Weiterhin hat sich herausgestellt, dass es von Vorteil ist, wenn die langkettigen Fettalkohole, insbesondere Arachylalkohol (Eicosan-1-ol) und/oder Behenylalkohol (Docosan-1-ol), in bestimmten Mengenbereichen in dem erfindungsgemäßen Mittel enthalten sind. Bevorzugte erfindungsgemäße Mittel enthalten ein oder mehrere langkettige Fettalkohole (a) aus der Gruppe Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), Heneicosylalkohol (Heneicosan-1-ol), Behenylalkohol (Docosan-1-ol), Erucylalkohol ((13Z)-Docos-13-en-1-ol) und Brassidylalkohol ((13*E*)-Docosen-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - bezogen auf das Gesamtgewicht des anwendungsbereiten Mittels.
In einer ganz besonders bevorzugten Ausführungsform ist ein erfindungsgemäßes Mittel dadurch gekennzeichnet, dass es als Fettalkohol(e) Arachylalkohol (Eicosan-1-ol) und/oder Behenylalkohol (Docosan-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - bezogen auf das Gesamtgewicht des anwendungsbereiten Mittels - enthält.

In einer ganz besonders bevorzugten Ausführungsform ist ein erfindungsgemäßes Mittel dadurch gekennzeichnet, dass es als Fettalkohol(e) Arachylalkohol (Eicosan-1-ol) und/oder Behenylalkohol (Docosan-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - bezogen auf das Gesamtgewicht des anwendungsbereiten Mittels - enthält.
Neben den speziellen langkettigen Fettalkoholen mit einer Kettenlänge von mindestens 20 C-Atomen kann das erfindungsgemäße Mittel zusätzlich auch noch weitere, kürzerkettige Fettalkohole mit einer Kettenlänge von 12 bis 18 C-Atomen enthalten. Geeignete kürzerkettige Fettalkohole mit einer gesättigten C₁₂-C₁₈-Alkylkette sind beispielsweise Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol) und Octadecan-1-ol (Octadecylalkohol, Stearylalkohol). Ein geeigneter kürzerkettiger Fettalkohol mit einer ungesättigten C₁₂-C₁₈-Alkylkette ist beispielsweise (9Z)-Octadec-9-en-1-ol (Oleylalkohol).

Ganz besonders bevorzugt im Hinblick auf die Konsistenz und Applizierbarkeit der erfindungsgemäßen Produkte ist der Einsatz von verzweigten längerkettigen Alkoholen mit einer Kettenlänge von 12 bis 18 C-Atomen, wobei sich Hexeyldecanol als besonders geeignet erwiesen hat. Erfindungsgemäß besonders bevorzugte Mittel sind dadurch gekennzeichnet, dass sie - bezogen auf ihr Gewicht - 2 bis 10 Gew.-%, weiter bevorzugt 3,5 bis 8 Gew.-%, besonders bevorzugt 4 bis 7 Gew.-% und insbesondere 5 bis 6,5 Gew.-% verzweigte längerkettige Alkohole mit einer Kettenlänge von 12 bis 18 C-Atomen, vorzugsweise 2-Hexyl-decan-1-ol, enthalten.

Besonders bevorzugt ist der Einsatz spezieller Polymere, um die physikalische und chemische Stabilität der erfindungsgemäßen Mittel weiter zu erhöhen. Bevorzugte erfindungsgemäße Mittel enthalten hierzu 0,05 bis 5 Gew.-% Polymer(e) aus der Gruppe der
i. Copolymere von Ethylen/Propylen/Styrol,
ii. Copolymere von Butylen/Ethylen/Styrol,
iii. Copolymere von Butylen/Propylen/Styrol
enthält

Vorzugsweise sind die genannten Copolymere keine Copolymere, in denen die Monomereinheiten statistisch verteilt sind, sondern Blockcoplymere, besonders bevorzugt Diblockcopolymere bzw. Triblockcopolymere. Solche Blockcopolymere weisen dann "harte" Segmente aus Polystyrol und "weiche" Segmente aus Ethylen/Propylen bzw. Ethylen/Butylen bzw. Propylen/Butylen auf. Die einzelnen Blöcke können dabei 10 bis 10000, vorzugsweise 50 bis 5000 und insbesondere 100 bis 500 Monomere umfassen. Bevorzugte Diblockcopolymere sind Styrol-Ethylenpropylen (S-EP) und Styrol-Ethylenbutylen (S-EB); bevorzugte Triblockcoplymere sind Styrol-Ethylenpropylen-Styrol (S-EP-S) und Styrol-Ethylenbutylen-Styrol (S-EB-S). Erfindungsgemäß besonders bevorzugt ist es, Mischungen von Diblock- und Triblock-Copolymeren einzusetzen, wobei sich Mischungen von Styrol-Ethylenpropylen (S-EP) und Styrol-Ethylenpropylen-Styrol (S-EP-S) als besonders bevorzugt herausgestellt haben. Ganz besonders bevorzugt beträgt hierbei der Anteil an Diblockcopolymeren 10 bis 90 Gew.-% und der Anteil an Triblockcopolymeren 90 bis 10 Gew.-% - jeweils bezogen auf das Gewicht der Polymermischung.

Erfindungsgemäß bevorzugte Mittel sind dadurch gekennzeichnet, dass sie 0,1 bis 4 Gew.-%, vorzugsweise 0,15 bis 3 Gew.-%, weiter bevorzugt 0,2 bis 2,5 Gew.-%, besonders bevorzugt 0,25 bis 2 Gew.-%, weiter bevorzugt 0,3 bis 1,5 Gew.-% und insbesondere 0,35 bis 0,75 Gew.-% Copolymere von Ethylen/Propylen/Styrol enthalten.

Erfindungsgemäß besonders bevorzugte Mittel sind dadurch gekennzeichnet, dass sie 0,1 bis 4 Gew.-%, vorzugsweise 0,15 bis 3 Gew.-%, weiter bevorzugt 0,2 bis 2,5 Gew.-%, besonders bevorzugt 0,25 bis 2 Gew.-%, weiter bevorzugt 0,3 bis 1,5 Gew.-% und insbesondere 0,35 bis 0,75 Gew.-% Diblock-Copolymere von Ethylen/Propylen/Styrol (S-EP) enthalten.

Erfindungsgemäß ebenfalls besonders bevorzugte Mittel sind dadurch gekennzeichnet, dass sie 0,1 bis 4 Gew.-%, vorzugsweise 0,15 bis 3 Gew.-%, weiter bevorzugt 0,2 bis 2,5 Gew.-%, besonders bevorzugt 0,25 bis 2 Gew.-%, weiter bevorzugt 0,3 bis 1,5 Gew.-% und insbesondere 0,35 bis 0,75 Gew.-% Triblock-Copolymere von Ethylen/Propylen/Styrol (S-EP-S) enthalten.

Besonders bevorzugte Diblock-Copolymere von Ethylen/Propylen/Styrol (S-EP) lassen sich durch die Formel (I) beschreiben 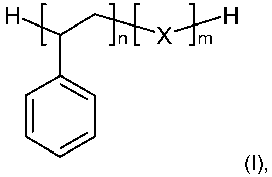 in der -[X]ₘ- für einen Block steht, der Ethylen- und Propylen-Monomereinheiten aufweist, die als Block oder statistisch verteilt vorliegen können, wobei m für eine Zahl von 10 bis 10000, vorzugsweise von 50 bis 5000 und insbesondere von 100 bis 500 steht und sich auf die Gesamtzahl an Ethylen- und Propylen-Monomereinheiten im Block bezieht und n für eine Zahl von 10 bis 10000, vorzugsweise von 50 bis 5000 und insbesondere von 100 bis 500 steht.

Besonders bevorzugte Triblock-Copolymere von Ethylen/Propylen/Styrol (S-EP-S) lassen sich durch die Formel (II) beschreiben 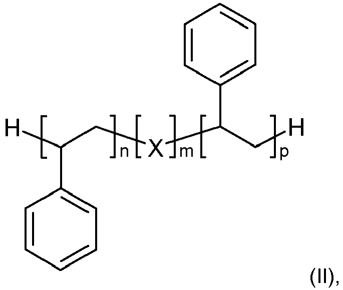 in der -[X]ₘ- für einen Block steht, der Ethylen- und Propylen-Monomereinheiten aufweist, die als Block oder statistisch verteilt vorliegen können, wobei m für eine Zahl von 10 bis 10000, vorzugsweise von 50 bis 5000 und insbesondere von 100 bis 500 steht und sich auf die Gesamtzahl an Ethylen- und Propylen-Monomereinheiten im Block bezieht und n und p unabhängig voneinander für eine Zahl von 10 bis 10000, vorzugsweise von 50 bis 5000 und insbesondere von 100 bis 500 stehen.

Erfindungsgemäß bevorzugte Mittel sind dadurch gekennzeichnet, dass sie 0,1 bis 4 Gew.-%, vorzugsweise 0,15 bis 3 Gew.-%, weiter bevorzugt 0,2 bis 2,5 Gew.-%, besonders bevorzugt 0,25 bis 2 Gew.-%, weiter bevorzugt 0,3 bis 1,5 Gew.-% und insbesondere 0,35 bis 0,75 Gew.-% Copolymere von Butylen/Ethylen/Styrol enthalten.

Erfindungsgemäß ebenfalls besonders bevorzugte Mittel sind dadurch gekennzeichnet, dass sie 0,1 bis 4 Gew.-%, vorzugsweise 0,15 bis 3 Gew.-%, weiter bevorzugt 0,2 bis 2,5 Gew.-%, besonders bevorzugt 0,25 bis 2 Gew.-%, weiter bevorzugt 0,3 bis 1,5 Gew.-% und insbesondere 0,35 bis 0,75 Gew.-% Diblock-Copolymere von Butylen/Ethylen/Styrol (S-EB) enthalten.

Erfindungsgemäß ebenfalls besonders bevorzugte Mittel sind dadurch gekennzeichnet, dass sie 0,1 bis 4 Gew.-%, vorzugsweise 0,15 bis 3 Gew.-%, weiter bevorzugt 0,2 bis 2,5 Gew.-%, besonders bevorzugt 0,25 bis 2 Gew.-%, weiter bevorzugt 0,3 bis 1,5 Gew.-% und insbesondere 0,35 bis 0,75 Gew.-% Triblock-Copolymere von Butylen/Ethylen/Styrol (S-EB-S) enthalten.

Besonders bevorzugte Diblock-Copolymere von Butylen/Ethylen/Styrol (S-EB) lassen sich durch die Formel (III) beschreiben 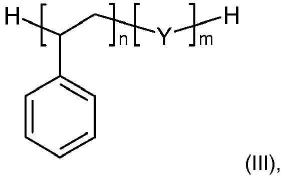 in der -[Y]ₘ- für einen Block steht, der Ethylen- und Butylen-Monomereinheiten aufweist, die als Block oder statistisch verteilt vorliegen können, wobei m für eine Zahl von 10 bis 10000, vorzugsweise von 50 bis 5000 und insbesondere von 100 bis 500 steht und sich auf die Gesamtzahl an Ethylen- und Butylen-Monomereinheiten im Block bezieht und n für eine Zahl von 10 bis 10000, vorzugsweise von 50 bis 5000 und insbesondere von 100 bis 500 steht.

Besonders bevorzugte Triblock-Copolymere von Butylen/Ethylen/Styrol (S-EB-S) lassen sich durch die Formel (IV) beschreiben 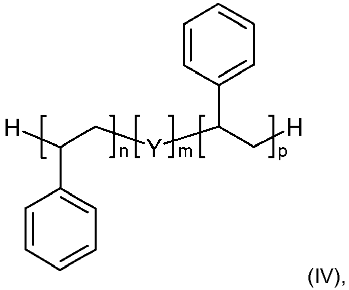 in der -[Y]ₘ- für einen Block steht, der Ethylen- und Butylen-Monomereinheiten aufweist, die als Block oder statistisch verteilt vorliegen können, wobei m für eine Zahl von 10 bis 10000, vorzugsweise von 50 bis 5000 und insbesondere von 100 bis 500 steht und sich auf die Gesamtzahl an Ethylen- und Butylen-Monomereinheiten im Block bezieht und n und p unabhängig voneinander für eine Zahl von 10 bis 10000, vorzugsweise von 50 bis 5000 und insbesondere von 100 bis 500 stehen.

Weiterhin können die Blondiermittel Alkalisierungsmittel enthalten. Bevorzugte Alkalisierungsmittel sind beispielsweise Ammoniak, Alkanolamine, basischen Aminosäuren, sowie anorganischen Alkalisierungsmittel wie (Erd-)Alkalimetallhydroxide, (Erd-)Alkalimetallmetasilikate, (Erd-) Alkalimetallphosphate und (Erd-)Alkalimetallhydrogenphosphate. Als Metallionen dienen bevorzugt Lithium, Natrium und/oder Kalium. Insbesondere bevorzugtes Alkalisierungsmittel ist Ammoniak. Erfindungsgemäß einsetzbare, anorganische Alkalisierungsmittel sind bevorzugt ausgewählt aus Natriumhydroxid, Kaliumhydroxid, Calciumhydroxid, Bariumhydroxid, Natriumphosphat, Kaliumphosphat, Natriumsilicat, Kaliumsilicat, Magnesiumsilicat, Natriumcarbonat und Kaliumcarbonat. Besonders bevorzugt sind Natriumhydroxid und/oder Kaliumhydroxid.

Es hat sich als bevorzugt herausgestellt, in den erfindungsgemäßen Zusammensetzungen Metasilikate einsetzen. Diese steigern die Bleichwirkung bei gleichzeitig verringerter Schädigung der keratinischen Faser. Hier haben sich bevorzugt (Erd-)Alkalimetallmetasilikate, besonders bevorzugt Alkalimetallmetasilikate und insbesondere Natriummetasilikat als geeignet erwiesen. Erfindungsgemäß bevorzugte Mittel enthalten daher - bezogen auf ihr Gewicht - 5 bis < 10 Gew.-%, vorzugsweise 6 bis < 9,5 Gew.-%, weiter bevorzugt 6,5 bis < 9 Gew.-%, besonders bevorzugt 7 bis < 8,5 Gew.-% und insbesondere 7,5 bis < 8 Gew.-% (Erd-)Alkalimetallmetasilikate, bevorzugt Alkalimetallmetasilikate und insbesondere Natriummetasilikat.

Erfindungsgemäß einsetzbare Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Insbesondere bevorzugte Alkanolamine sind Monoethanolamin, 2-Amino-2-methyl-propanol und Triethanolamin.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, L-Ornithin, D- Ornithin, D/L- Ornithin, L-Histidin, D-Histidin und/oder D/L-Histidin. Besonders bevorzugt werden L-Arginin, D-Arginin und/oder D/L-Arginin als ein Alkalisierungsmittel im Sinne der Erfindung eingesetzt.

Manche Kunden empfinden die intensive Geruchsbildung von Ammoniak belästigend oder störend. Obwohl Ammoniak ein bevorzugtes Alkalisierungsmittel ist, können daher anwendungsbereite Zubereitungen erfindungsgemäß bevorzugt sein, die frei von Ammoniak sind. Bevorzugte Alkalisierungsmittel für Zubereitungen, die frei von Ammoniak sind, sind Monoethanolamin, 2-Amino-2-methyl-propanol und Triethanolamin.

Wenn die anwendungsbereiten Mischungen Alkalisierungsmittel enthalten, sind Zubereitungen erfindungsgemäß bevorzugt, die Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Die erfindungsgemäßen Zusammensetzungen können zusätzlich mindestens einen weiteren Bleichverstärker, der von den anorganischen Persalzen verschieden ist, enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Ein zweiter Gegenstand der Erfindung ist ein Verfahren zur Farbveränderung keratinischer Fasern, bei dem mindestens zwei getrennt voneinander verpackte Zubereitungen (A) und (B), von welchen Zubereitung (A) mindestens ein Persulfat und Zubereitung (B) mindestens ein Oxidationsmittel enthält, zu einer Anwendungsmischung vermischt werden, diese auf die Fasern aufgetragen wird und nach einer Einwirkungszeit wieder abgespült wird, dadurch gekennzeichnet, dass Zubereitung (A)
a) 20 bis 75 Gew.-% Öl(e);
b) 0,05 bis 5 Gew.-% Xanthan,
c) 1 bis 70 Gew.-% Peroxidisulfat(e),
d) 1 bis 15 Gew.-% Cetearylakohol.
enthält.

Die anwendungsbereiten Mittel werden unmittelbar vor der Anwendung auf dem Haar durch Mischen der zwei Zubereitungen (A) und (B) und gegebenenfalls einer dritten Zubereitung (C) und/oder weiteren Zubereitungen hergestellt. Bei anwendungsbereiten Mitteln, die aus mehr als zwei Zubereitungen zu einer fertigen Anwendungsmischung vermischt werden, kann es unerheblich sein, ob zunächst zwei Zubereitungen miteinander vermischt werden und anschließend die dritte Zubereitung zugegeben und untergemischt wird, oder ob alle Zubereitungen gemeinsam zusammengeführt und anschließend vermischt werden. Das Vermischen kann durch Verrühren in einer Schale oder einem Becher erfolgen oder durch Schütteln in einem verschließbaren Behälter.

Der Begriff "unmittelbar" ist dabei als Zeitraum von wenigen Sekunden bis eine Stunde, vorzugsweise bis 30 min, insbesondere bis 15 min zu verstehen.

Die erfindungsgemäßen Mittel werden in einem Verfahren zum Aufhellen von keratinischen Fasern, insbesondere menschlichen Haaren, angewendet, bei dem das Mittel auf die keratinhaltigen Fasern aufgebracht, bei einer Temperatur von Raumtemperatur bis 45 °C für eine Einwirkdauer von 10 bis 60 Minuten auf der Faser belassen und anschließend mit Wasser wieder ausgespült oder mit einem Shampoo ausgewaschen wird.

Bevorzugt beträgt die Einwirkzeit der anwendungsbereiten Aufhellmittel 10 bis 60 min, insbesondere 15 bis 50 min, besonders bevorzugt 20 bis 45 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Bevorzugt liegt die Temperatur während der Einwirkzeit zwischen 20°C und 40°C, insbesondere zwischen 25°C und 38°C. Die Aufhellmittel ergeben bereits bei physiologisch verträglichen Temperaturen von unter 45°C gute Blondier- und Aufhellergebnisse.

Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensidhaltigen Träger besitzt.

Die bevorzugten Ausführungsformen des ersten Erfindungsgegenstands gelten mutatis mutandis auch für den zweiten Erfindungsgegenstand.

## Patentansprüche

1. Mittel zum Aufhellen keratinischer Fasern, enthaltend - bezogen auf sein Gewicht -
a) 20 bis 75 Gew.-% Öl(e);
b) 0,05 bis 5 Gew.-% Xanthan,
c) 1 bis 70 Gew.-% Peroxidisulfat(e),
d) 1 bis 15 Gew.-% Cetearylakohol.

2. Mittel nach Anspruch 1, **dadurch gekennzeichnet, dass** es 22,5 bis 70 Gew.-%, vorzugsweise 25 bis 65 Gew.-%, weiter bevorzugt 27,5 bis 60 Gew.-%, besonders bevorzugt 30 bis 55 Gew.-% und insbesondere 32,5 bis 50 Gew.-% Öl(e) aus der Gruppe Paraffinöl, Polyisobuten, der Alkylbenzoate, Isopropylpalmitat, Isohexadekan, Isododecan, Isononyl-Isononanoat enthält.

3. Mittel nach einem der Ansprüche 1 oder 2, **dadurch gekennzeichnet, dass** es 20 bis 60 Gew.-%, vorzugsweise 22,5 bis 55 Gew.-%, weiter bevorzugt 25 bis 50 Gew.-%, besonders bevorzugt 27,5 bis 45 Gew.-% und insbesondere 30 bis 40 Gew.-% Paraffinöl enthält.

4. Mittel nach einem der Ansprüche 1 bis 3, **dadurch gekennzeichnet, dass** es 0,1 bis 5 Gew.-%, vorzugsweise 0,5 bis 4 Gew.-%, weiter bevorzugt 1 bis 3 Gew.-%, besonders bevorzugt 1,25 bis 2,5 Gew.-% und insbesondere 1,5 bis 2 Gew.-% Xanthan enthält.

5. Mittel nach einem der Ansprüche 1 bis 4, **dadurch gekennzeichnet, dass** es 5 bis 43,5 Gew.-%, vorzugsweise 10 bis 43 Gew.-%, weiter bevorzugt 15 bis 42,5 Gew.-%, besonders bevorzugt 20 bis 42 Gew.-% und insbesondere 30 bis 41 Gew.-% Kaliumperoxidisulfat enthält.

6. Mittel nach einem der Ansprüche 1 bis 5, **dadurch gekennzeichnet, dass** es 0 bis < 2,5 Gew.-%, vorzugsweise 0 bis < 1 Gew.-%, weiter bevorzugt 0 bis < 0,5 Gew.-%, besonders bevorzugt 0 bis < 0,1 Gew.-% und insbesondere 0 Gew.-% Peroxidisulfate aus der Gruppe Natriumperoxidisulfat und/oder Ammoniumperoxidisulfat enthält.

7. Mittel nach einem der Ansprüche 1 bis 6, **dadurch gekennzeichnet, dass** es es 1,5 bis 12,5 Gew.-%, vorzugsweise 2 bis 10 Gew.-%, weiter bevorzugt 3,5 bis 8 Gew.-%, besonders bevorzugt 4 bis 7 Gew.-% und insbesondere 5 bis 6,5 Gew.-% Cetearlyalkohol enthält.

8. Mittel nach einem der Ansprüche 1 bis 7, **dadurch gekennzeichnet, dass** es ein oder mehrere langkettige Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11Z*,*14*Z*)-Eicosa-5,8,11,14-tetraen-1-ol), Heneicosylalkohol (Heneicosan-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,3 bis 3,4 Gew.-%, bevorzugt von 0,4 bis 2,6 Gew.-%, weiter bevorzugt von 0,5 bis 1,8 Gew.-% und besonders bevorzugt von 0,6 bis 0,9 Gew.-% - enthält.

9. Mittel nach einem der Ansprüche 1 bis 8, **dadurch gekennzeichnet, dass** es - bezogen auf sein Gewicht - 2 bis 10 Gew.-%, weiter bevorzugt 3,5 bis 8 Gew.-%, besonders bevorzugt 4 bis 7 Gew.-% und insbesondere 5 bis 6,5 Gew.-% verzweigte längerkettige Alkohole mit einer Kettenlänge von 12 bis 18 C-Atomen, vorzugsweise 2-Hexyl-decan-1-ol, enthält.

10. Mittel nach einem der Ansprüche 1 bis 9, **dadurch gekennzeichnet, dass** es - bezogen auf ihr Gewicht - 5 bis < 10 Gew.-%, vorzugsweise 6 bis < 9,5 Gew.-%, weiter bevorzugt 6,5 bis < 9 Gew.-%, besonders bevorzugt 7 bis < 8,5 Gew.-% und insbesondere 7,5 bis < 8 Gew.-% (Erd-)Alkalimetallmetasilikate, bevorzugt Alkalimetallmetasilikate und insbesondere Natriummetasilikat enthält.

11. Mittel nach einem der Ansprüche 1 bis 10, **dadurch gekennzeichnet, dass** es 0,05 bis 5 Gew.-% Polymer(e) aus der Gruppe der
i. Copolymere von Ethylen/Propylen/Styrol,
ii. Copolymere von Butylen/Ethylen/Styrol,
iii. Copolymere von Butylen/Propylen/Styrol
enthält.

12. Mittel nach einem der Ansprüche 1 bis 11, **dadurch gekennzeichnet, dass** es 0,1 bis 4 Gew.-%, vorzugsweise 0,15 bis 3 Gew.-%, weiter bevorzugt 0,2 bis 2,5 Gew.-%, besonders bevorzugt 0,25 bis 2 Gew.-%, weiter bevorzugt 0,3 bis 1,5 Gew.-% und insbesondere 0,35 bis 0,75 Gew.-% Copolymere von Ethylen/Propylen/Styrol enthält.

13. Verfahren zur Farbveränderung keratinischer Fasern, bei dem mindestens zwei getrennt voneinander verpackte Zubereitungen (A) und (B), von welchen Zubereitung (A) mindestens ein Persulfat und Zubereitung (B) mindestens ein Oxidationsmittel enthält, zu einer Anwendungsmischung vermischt werden, diese auf die Fasern aufgetragen wird und nach einer Einwirkungszeit wieder abgespült wird, **dadurch gekennzeichnet, dass** Zubereitung (A)
a) 20 bis 75 Gew.-% Öl(e);
b) 0,05 bis 5 Gew.-% Xanthan,
c) 1 bis 70 Gew.-% Peroxidisulfat(e),
d) 1 bis 15 Gew.-% Cetearylakohol.
enthält.

## Claims

1. An agent for lightening keratin fibers, containing, based on its weight,
a) from 20 to 75 wt.% oil(s);
b) from 0.05 to 5 wt.% xanthan gum;
c) from 1 to 70 wt.% peroxydisulfate(s);
d) from 1 to 15 wt.% cetearyl alcohol.

2. The agent according to claim 1, **characterized in that** it contains from 22.5 to 70 wt.%, preferably from 25 to 65 wt.%, more preferably from 27.5 to 60 wt.%, particularly preferably from 30 to 55 wt.%, and in particular from 32.5 to 50 wt.% oil(s) from the group consisting of paraffin oil, polyisobutene, alkyl benzoates, isopropyl palmitate, isohexadecane, isododecane, and isononyl isononanoate.

3. The agent according to one of claims 1 or 2, **characterized in that** it contains from 20 to 60 wt.%, preferably from 22.5 to 55 wt.%, more preferably from 25 to 50 wt.%, particularly preferably from 27.5 to 45 wt.%, and in particular from 30 to 40 wt.% paraffin oil:

4. The agent according to one of claims 1 to 3, **characterized in that** it contains from 0.1 to 5 wt.%, preferably from 0.5 to 4 wt.%, more preferably from 1 to 3 wt.%, particularly preferably from 1.25 to 2.5 wt.%, and in particular from 1.5 to 2 wt.% xanthan gum.

5. The agent according to one of claims 1 to 4, **characterized in that** it contains from 5 to 43.5 wt.%, preferably from 10 to 43 wt.%, more preferably from 15 to 42.5 wt.%, particularly preferably from 20 to 42 wt.%, and in particular from 30 to 41 wt.% potassium peroxydisulfate.

6. The agent according to one of claims 1 to 5, **characterized in that** it contains from 0 to < 2.5 wt.%, preferably from 0 to < 1 wt.%, more preferably from 0 to < 0.5 wt.%, particularly preferably from 0 to < 0.1 wt.%, and in particular 0 wt.% peroxydisulfates from the group consisting of sodium peroxydisulfate and/or ammonium peroxydisulfate.

7. The agent according to one of claims 1 to 6, **characterized in that** it contains from 1.5 to 12.5 wt.%, preferably from 2 to 10 wt.%, more preferably from 3.5 to 8 wt.%, particularly preferably from 4 to 7 wt.%, and in particular from 5 to 6.5 wt.% cetearyl alcohol.

8. The agent according to one of claims 1 to 7, **characterized in that** it contains one or more long-chain fatty alcohols from the group consisting of arachyl alcohol (eicosan-1-ol), gadoleyl alcohol ((9Z)-eicos-9-en-1-ol), arachidonic alcohol ((5Z,8Z,11Z,14Z)-eicosa-5,8,11,14-tetraen-1-ol), heneicosyl alcohol (heneicosan-1-ol), behenyl alcohol (docosan-1-ol), erucyl alcohol ((13Z)-docos-13-en-1-ol), and brassidyl alcohol ((13E)-docosen-1-ol) in a total amount of from 0.3 to 3.4 wt.%, preferably from 0.4 to 2.6 wt.%, more preferably from 0.5 to 1.8 wt.% and particularly preferably from 0.6 to 0.9 wt.%.

9. The agent according to one of claims 1 to 8, **characterized in that** it contains, based on its weight, from 2 to 10 wt.%, more preferably from 3.5 to 8 wt.%, particularly preferably from 4 to 7 wt.%, and in particular from 5 to 6.5 wt.% branched longer chain alcohols having a chain length of 12 to 18 C atoms, preferably 2-hexyl-decan-1-ol.

10. The agent according to one of claims 1 to 9, **characterized in that** it contains, based on its weight, from 5 to < 10 wt.%, preferably from 6 to < 9.5 wt.%, more preferably from 6.5 to < 9 wt.%, particularly preferably from 7 to < 8.5 wt.%, and in particular from 7.5 to < 8 wt.% alkaline earth metal metasilicates or alkali metal metasilicates, preferably alkali metal metasilicates, and in particular sodium metasilicate.

11. The agent according to one of claims 1 to 10, **characterized in that** it contains from 0.05 to 5 wt.% polymer(s) from the group consisting of the
i. copolymers of ethylene/propylene/styrene,
ii. copolymers of butylene/ethylene/styrene, and
iii. copolymers of butylene/propylene/styrene.

12. The agent according to one of claims 1 to 11, **characterized in that** it contains from 0.1 to 4 wt.%, preferably from 0.15 to 3 wt.%, more preferably from 0.2 to 2.5 wt.%, particularly preferably from 0.25 to 2 wt.%, more preferably from 0.3 to 1.5 wt.%, and in particular from 0.35 to 0.75 wt.% copolymers of ethylene/propylene/styrene.

13. A method for changing the color of keratin fibers, in which method at least two preparations (A) and (B) packaged separately from each other, of which preparation (A) contains at least one persulfate and preparation (B) contains at least one oxidizing agent, are mixed to form an application mixture, said mixture is applied to the fibers, and is rinsed off again after a contact time, **characterized in that** preparation (A) contains
a) from 20 to 75 wt.% oil(s);
b) from 0.05 to 5 wt.% xanthan gum;
c) from 1 to 70 wt.% peroxydisulfate(s);
d) from 1 to 15 wt.% cetearyl alcohol.

## Revendications

1. Agent pour l'éclaircissement de fibres kératiniques, contenant- par rapport à son poids -
a) de 20 à 75 % en poids d'huile(s),
b) de 0,05 à 5 % en poids de gomme xanthane,
c) de 1 à 70 % en poids de peroxodisulfate(s),
d) de 1 à 15 % en poids d'alcool cétéarylique.

2. Agent selon la revendication 1, **caractérisé en ce qu'**il contient de 22,5 à 70 % en poids, de préférence de 25 à 65 % en poids, de façon plus préférable de 27,5 à 60 % en poids, de façon particulièrement préférée de 30 à 55 % en poids et en particulier de 32,5 à 50 % en poids d'huile(s) issues du groupe constitué par l'huile de paraffine, le polyisobutène, l'alkylbenzoate, l'isopropylpalmitate, l'isohexadécane, l'isododécane et l'isononyl-isononanoate.

3. Agent selon l'une des revendications 1 ou 2, **caractérisé en ce qu'**il contient de 20 à 60 % en poids, de préférence de 22,5 à 55 % en poids, de façon plus préférable de 25 à 50 % en poids, de façon particulièrement préférée de 27,5 à 45 % en poids et en particulier de 30 à 40 % en poids d'huile de paraffine.

4. Agent selon l'une des revendications 1 à 3, **caractérisé en ce qu'**il contient de 0,1 à 5 % en poids, de préférence de 0,5 à 4 % en poids, de façon plus préférable de 1 à 3 % en poids, de façon particulièrement préférée de 1,25 à 2,5 % en poids et en particulier de 1,5 à 2 % en poids de gomme xanthane.

5. Agent selon l'une des revendications 1 à 4, **caractérisé en ce qu'**il contient de 5 à 43,5 % en poids, de préférence de 10 à 43 % en poids, de façon plus préférable de 15 à 42,5 % en poids, de façon particulièrement préférée de 20 à 42 % en poids et en particulier de 30 à 41 % en poids de peroxodisulfate de potassium.

6. Agent selon l'une des revendications 1 à 5, **caractérisé en ce qu'**il contient de 0 à < 2,5 % en poids, de préférence de 0 à < 1 % en poids, de façon plus préférable de 0 à < 0,5 % en poids, de façon particulièrement préférée de 0 à < 0,1 % en poids et en particulier 0 % en poids de peroxodisulfate du groupe constitué par le peroxodisulfate de sodium et/ou le peroxodisulfate d'ammonium.

7. Agent selon l'une des revendications 1 à 6, **caractérisé en ce qu'**il contient de 1,5 à 12,5 % en poids, de préférence de 2 à 10 % en poids, de façon plus préférable de 3,5 à 8 % en poids, de façon particulièrement préférée de 4 à 7 % en poids et en particulier de 5 à 6,5 % en poids d'alcool cétéarylique.

8. Agent selon l'une des revendications 1 à 7, **caractérisé en ce qu'**il contient un ou plusieurs alcools gras à chaîne longue du groupe constitué par l'alcool arachidylique (Eicosan-1-ol), l'alcool gadoléique ((9Z)-Eicos-9-en-1-ol), l'alcool arachidonique ((5Z,8Z,11Z,14Z)-Eicosa-5,8,11,14-tetraen-1-ol),Heneicosylalkohol (Heneicosan-1-ol), l'alcool béhénylique (Docosan-1-ol), l'alcool érucylique ((13Z)-Docos-13-en-1-ol) et l'alcool brassidylique ((13E)-Docosen-1-ol) en une quantité totale allant de 0,3 à 3,4 % en poids, de préférence de 0,4 à 2,6 % en poids, de façon plus préférable de 0,5 à 1,8 % en poids et de façon particulièrement préférée de 0,6 à 0,9 % en poids.

9. Agent selon l'une des revendications 1 à 8, **caractérisé en ce qu'**il contient - par rapport à son poids - de 2 à 10 % en poids, de façon plus préférable de 3,5 à 8 % en poids, de façon particulièrement préférée de 4 à 7 % en poids et en particulier de 5 à 6,5 % en poids d'alcools à chaîne longue ramifiés, avec une longueur de chaîne allant de 12 à 18 atomes de carbone, de préférence le 2-hexyl-décan-1-ol.

10. Agent selon l'une des revendications 1 à 9, **caractérisé en ce qu'**il contient - par rapport à son poids - de 5 à < 10 % en poids, de préférence de 6 à < 9,5 % en poids, de façon plus préférable de 6,5 à < 9 % en poids, de façon particulièrement préférée de 7 à < 8,5 % en poids et en particulier de 7,5 à < 8 % en poids de métasilicate de métal alcalin(o-terreux), de préférence de métasilicate de métal alcalin et en particulier de métasilicate de sodium.

11. Agent selon l'une des revendications 1 à 10, **caractérisé en ce qu'**il contient de 0,05 à 5 % en poids de polymère(s) du groupe des
i. copolymères d'éthylène/propylène/styrol,
ii. copolymères de butylène/éthylène/styrol,
iii. copolymères de butylène/propylène/styrol.

12. Agent selon l'une des revendications 1 à 11, **caractérisé en ce qu'**il contient de 0,1 à 4 % en poids, de préférence de 0,15 à 3 % en poids, de façon plus préférable de 0,2 à 2,5 % en poids, de façon particulièrement préférée de 0,25 à 2 % en poids, de façon encore plus préférée de 0,3 à 1,5 % en poids et en particulier de 0,35 à 0,75 % en poids de copolymères d'éthylène/propylène/styrol.

13. Procédé de coloration des fibres kératiniques, dans lequel au moins deux préparations (A) et (B) conditionnées séparément l'une de l'autre, dans lesquelles la préparation (A) contient au moins un persulfate et la préparation (B) contient au moins un agent oxydant, sont mélangées pour obtenir un mélange à appliquer, lequel est déposé sur les fibres puis rincé après un temps de pose, **caractérisé en ce que** la préparation (A) contient
a) de 20 à 75 % en poids d'huile(s),
b) de 0,05 à 5 % en poids de gomme xanthane,
c) de 1 à 70 % en poids de peroxodisulfate(s),
d) de 1 à 15 % en poids d'alcool cétéarylique.
